# EUROPÄISCHE PATENTSCHRIFT

(11) **EP 1 645 308 B1**
(45) Veröffentlichungstag und Bekanntmachung des Hinweises auf die Patenterteilung: **16.06.2010**
(21) Anmeldenummer: 04023784.4
(22) Anmeldetag: 06.10.2004
(51) Int. Cl.: A61N 5/10, G06F 19/00

(54) **Verfahren zur Planung einer Gewebebehandlung**
Method for planning the treatment of tissue
Méthode de planification d'un traitement de tissue

(43) Veröffentlichungstag der Anmeldung: 12.04.2006
(73) Patentinhaber: BrainLAB AG, 85622 Feldkirchen (DE)
(72) Erfinder: Hartlep, Andreas, Dr., 83629 Naring (DE); Pedain, Christoph, Dr., 81667 München (DE)
(74) Vertreter: Schwabe - Sandmair - Marx

(56) Entgegenhaltungen:
- US-A- 6 011 563
- US-A- 6 095 975
- US-A1- 2003 114 751
- HAMACHER K A ET AL: "Theoretical estimation of absorbed dose to organs in radioimmunotherapy using radionuclides with multiple unstable daughters" MEDICAL PHYSICS, AMERICAN INSTITUTE OF PHYSICS. NEW YORK, US, Bd. 28, Nr. 9, September 2001 (2001-09), Seiten 1857-1874, XP012011572 ISSN: 0094-2405
- FURHANG E E ET AL: "A MONTE CARLO APPROACH TO PATIENT-SPECIFIC DOSIMETRY" MEDICAL PHYSICS, AMERICAN INSTITUTE OF PHYSICS. NEW YORK, US, Bd. 23, Nr. 9, September 1996 (1996-09), Seiten 1523-1529, XP000640764 ISSN: 0094-2405
- AHMAD S U ET AL: "A new recursive algorithm for optimization in intensity modulated radiotherapy" ENGINEERING IN MEDICINE AND BIOLOGY SOCIETY, 2000. PROCEEDINGS OF THE 22ND ANNUAL INTERNATIONAL CONFERENCE OF THE IEEE 23-28 JULY 2000, PISCATAWAY, NJ, USA,IEEE, Bd. 4, 23. Juli 2000 (2000-07-23), Seiten 3100-3103, XP010531299 ISBN: 0-7803-6465-1

## Beschreibung

Die vorliegende Erfindung bezieht sich auf ein Verfahren zur Verbesserung der Planung einer Gewebebehandlung, insbesondere auf die Verbesserung der kombinierten Behandlung eines Gewebes durch Verabreichung einer Substanz in Kombination mit der Abgabe oder Verabreichung von Energie.

Bei der Behandlung eines Gewebes, insbesondere bei der Behandlung von Tumoren im Gehirn, der Prostata, des Körperstamms, der Leber oder der Brust, kann eine feste, flüssige oder gasförmige und zum Beispiel radioaktive Substanz in das Gewebe oder in die Nähe des Gewebes gebracht werden, um zum Beispiel eine so genannte Nahfeldbestrahlung des erkrankten Gewebes durchzuführen, so dass ein Tumor mittels eingebrachter radioaktiver Festkörper, Flüssigkeiten oder Gase, welche zum Beispiel β-Strahlung abgeben, zerstört werden kann.

Alternativ zu diesen Brachytherapieverfahren, bei welchen eine Bestrahlung eines Gewebes durch eine in die Nähe des zu bestrahlenden Gewebes eingebrachte radioaktive Substanz durchgeführt wird, kann auch ein radiotherapeutisches oder radiochirurgisches Verfahren durchgeführt werden, wobei eine Bestrahlung des Gewebes zum Beispiel durch eine um den Körper geführte Strahlungsquelle durchgeführt wird. Dabei werden häufig Substanzen, sogenannte Sensitizer, in den Körper, insbesondere in den Bereich des zu behandelnden Gewebes, eingebracht, welcher zum Beispiel Tumorzellen empfindlicher bezüglich radioaktiven oder Lichtstrahlen oder auch Wärmeeinwirkung machen. Zur Behandlung eines Tumors werden gewöhnlich höhere Dosen dieser Sensitizer und höhere Strahlungsdosen verwendet, um mit einer möglichst hohen Wahrscheinlichkeit einen Großteil der Tumorzellen abtöten zu können. Jedoch kann die Verabreichung einer hohen Dosis an Sensitizern oder die Bestrahlung von Körpergewebe mit einer hohen Dosis radioaktiver Strahlung auch unerwünschte Wirkungen auf gesundes nicht zu behandelndes Gewebe haben.

Aus der US 5,735,814 ist die Infusion von Medikamenten in das Gehirn zur Behandlung von neurodegenerativen Funktionsstörungen mittels einer implantierbaren Pumpe und eines Katheters bekannt, wobei mittels der verabreichten Substanzen das Anregungsniveau der Neuronen im Gehirn verändert wird.

Die US 6,026,316 beschreibt die Überwachung der Verabreichung einer Substanz mittels Kernspintomographie.

Ein bei der Brachytherapie verwendbarer fester Stoff, welcher implantiert werden kann, ist aus der US 6,746,661 B2 bekannt.

Aus der US 6,011,563 ist ein Verfahren zur Tumor-Therapie mittels Laserlicht bekannt, wobei eine Simulation der PDT Behandlung durchgeführt wird.

Aus der US 6,095,975 ist ein Verfahren zum Bestimmen von optimalen Stellen zur Platzierung von radioaktiven Substanzen an einer kanzerogenen Stelle bekannt.

Aus HAMACHER K. A. et. al:" Theoretical estimation of absorbed dose to organs in radioimmunotherapy using radionuclides with multiple unstable daughters", MEDICAL PHYSICS, AMERICAN INSTITUTE OF PHYSICS, NEW YORK, US, Bd. 28, Nr. 9, September 2001 (2001-09), Seiten 1857-1874, XP012011572, ISSN: 0094-2405 ist eine theoretische Abschätzung einer durch Organe absorbierten Strahlendosis bei einer Radioimmunotherapie bekannt, bei welcher Radionuklide mit mehreren instabilen Tochterelementen verwendet werden, wobei ein Modell zur Beschreibung des Übergangs von einer Quelle in Blut bis zu einem Ziel verwendet wird.

Es ist eine Aufgabe der vorliegenden Erfindung ein Verfahren vorzuschlagen, welches eine zielgerichtete und die Gesundheit schonende Behandlung eines Gewebes ermöglicht.

Diese Aufgabe wird durch das in dem unabhängigen Anspruch definierte Verfahren gelöst. Vorteilhafte Ausführungsformen sind in den abhängigen Ansprüchen definiert.

Bei dem erfindungsgemäßen Verfahren wird die Verabreichung einer Substanz, insbesondere eines Sensitizers, wie zum Beispiel eines Radiosensitizers, Photosensitizers oder Thermosensitizers, zum Beispiel auch in Kombination mit der Verabreichung eines Desensitizers zum Beispiel an einer anderen Stelle des Körpers, simuliert und/oder geplant, indem basierend auf den Verabreichungs- oder Infusionsparametern der Substanz und/oder substanzspezifischer Parameter die Verteilung der Substanz im Körper simuliert wird.
Das Verfahren wird iterativ durchgeführt, wenn das simulierte Ergebnis der Auswirkung der Verabreichung der Substanz oder Energie auf Gewebe unterhalb eines vorgegebenen Mindesteffektes liegt.
Zusätzlich kann basierend auf der simulierten Verteilung der Substanz im Körper berechnet werden, welche Energie in welcher Form dem Körper zugeführt wird oder werden muss, um eine gewünschte Wirkung auf das Gewebe durch die verabreichte Substanz und die dem Körper oder Gewebe zugeführte Energie hervorzurufen, insbesondere um eine möglichst gezielte Zerstörung von Tumorzellen zu bewirken. Erfindungsgemäß wird der Benutzer durch die Erfindung anhand angezeigter Simulationsergebnisse oder Teilergebnisse dabei unterstützt, die Behandlung so zu optimieren, dass die gewünschte Wirkung mit erhöhter Wahrscheinlichkeit eintritt.

Die Substanz oder die Substanzen bzw. Wirkstoffe, welche zum Beispiel durch Infusion an einer oder mehreren Stellen an einen Körper verabreicht werden, kann zum Beispiel eine flüssige oder auch eine feste Substanz sein, welche sich zum Beispiel erst im Körper in Wechselwirkung mit einer Körperflüssigkeit oder einer zugeführten Flüssigkeit löst und kann auch radioaktive Elemente enthalten, welche beispielsweise mit anderen verabreichten Substanzen oder Fluiden wechselwirken können, um eine möglichst gerichtete oder gezielte Behandlung oder Zerstörung eines bestimmten und zum Beispiel vorgegebenen Gewebebereichs oder Gewebetyps zu ermöglichen.

Bezüglich der Ausgestaltung eines Verfahrens zur Planung einer Infusion und insbesondere der Verwendung von Patientendaten zur Planung oder Durchführung der Infusion wird auf die Lehre der EP 1 316 324 A1 der Anmelderin verwiesen. Insbesondere ist es vorteilhaft Patientendaten oder daraus gewonnene Patientenparameter zur Infusionsplanung zu verwenden, welche zum Beispiel mittels bekannter Kernspinresonanzverfahren (MRI), Computertomographie (CT)-Verfahren, Röntgen-, Ultraschall-Verfahren oder anderen Verfahren gewonnen wurden, welche es ermöglichen die räumliche Struktur eines Körpers und insbesondere die Gewebestruktur oder Gewebeeigenschaften zu erfassen und darzustellen und/oder funktionelle Daten wie zum Beispiel patientenspezifische Diffusions- oder Perfusionseigenschaften oder ―parameter zu gewinnen, so dass zum Beispiel ein oder mehrere geeignete Katheter aus einem vorgegebenen Satz von Kathetern zum Beispiel zusammen mit einer oder mehreren Punkten zur Infusion ausgewählt und unter Verwendung einer oder mehrerer dieser Daten hinsichtlich Anbringungsort und/oder Eindringtiefe positioniert werden. Ebenso wird vorteilhaft anhand einer oder mehrerer dieser Daten ein geeignetes Infusionsmittel ausgewählt oder die Zusammensetzung eines Infusionsmittels spezifiziert und zum Beispiel der zeitliche Verlauf des Abgabedruckes oder die Dauer der Verabreichung der Substanz berechnet.

Ist die Verteilung der an das Gewebe oder einen Körper zu verabreichenden Substanz im Körper simuliert worden, insbesondere unter Berücksichtigung von körperspezifischen Daten oder allgemeinen Wirkmechanismen, welche die Verteilung einer Substanz in einem Körper beeinflussen, wie zum Beispiel der Blut-Hirn-Schranke oder des Stoffwechsels, so kann festgestellt werden welchen Teilen des Körpers, in denen sich die durch Simulation ermittelte Menge oder Konzentration einer oder mehrerer verabreichter Substanzen befindet, Energie zugeführt werden soll. Zusätzlich kann basierend auf den Simulationsergebnissen oder Teilergebnissen berechnet werden, welche Wirkung die applizierte Energie auf das Gewebe haben wird. Insbesondere kann basierend auf der simulierenden Verteilung der Substanz(en) im Körper oder im Gewebe eine Verteilung der zu verabreichenden Energie berechnet werden, um in Kombination mit der im Körper befindlichen verabreichten Substanz eine möglichst große Wirkung zu erzielen. Beispielsweise kann berechnet werden welche Stelle oder welcher Bereich des Körpers oder Gewebes bestrahlt werden sollte, um zum Beispiel in Verbindung mit einem an dieser Stelle in einer bestimmten Konzentration vorliegenden Radiosensitizer eine genau dosierte oder dosierbare Wirkung zum Beispiel auf einen bestimmten Gewebebereich oder bestimmte Zelltypen zu haben. Weiterhin können zusätzliche Bestrahlungsparameter berechnet werden, wie zum Beispiel die Positionierung oder Führung einer in den Körper eingeführten oder außerhalb des Körpers angeordneten und um den Körper herum führbaren Strahlungsquelle. Ebenso ist es möglich, dass Energie nicht oder nicht nur in Form ionisierender Strahlung, sondern auch als Wärmeenergie oder Licht zugeführt wird, um mit einer verabreichten Substanz zu wechselwirken. Weiterhin soll im Sinne der Erfindung auch die gezielte oder gerichtete Zufuhr von Kälte zum Beispiel in Form einer kalten oder kühlenden Substanz oder Flüssigkeit als Energiezufuhr verstanden werden, welche in Verbindung mit einer in ein Gewebe eingebrachten Substanz eine Wirkung auf Gewebe und insbesondere eine Zerstörung eines bestimmten Zellentyps bewirken kann.

Vorzugsweise können Körper- oder Gewebeeigenschaften, wie zum Beispiel allgemeine oder personenspezifische Eigenschaften des Gehirns, zum Beispiel durch MRI, Ultraschall, CT, PET, SPECT, Biopsie oder anderen bekannte Messverfahren ermittelt werden, wobei unter Körper- oder Gewebeeigenschaften im Sinne der Erfindung Eigenschaften verstanden werden können, welche für die Verteilung oder chemische Zusammensetzung einer oder mehrerer verabreichter Substanzen relevant sind, wie zum Beispiel Diffusions- oder Perfusionseigenschaften, die Auswirkung des Stoffwechsels, oder die räumliche Ausbildung einer oder mehrerer Gewebestrukturen.

Bevorzugt wird die Auswirkung einer verabreichten Substanz auf das Gewebe bei der Simulation der Verteilung der Substanz im Körper oder Gewebe berücksichtigt, insbesondere eine Veränderung der Gewebedichte, der Veränderung des Anteils des Extrazellulärraums am Gesamtvolumen eines Gewebesteiles, der Sensitivität des Gewebes zum Beispiel bezüglich Wärme oder einer Strahlendosis, um zum Beispiel einen bestimmten Zellentyp abzutöten und um so das Absorptionsverhalten oder die Wechselwirkung mit der verabreichten Energie realitätsnah zu simulieren bzw. zu berücksichtigen.

Weiterhin kann die therapeutische Wirkung der Verabreichung einer oder mehrerer Substanzen in Kombination mit der Zufuhr von Energie simuliert werden, um als Simulationsergebnis die Auswirkung der Behandlung des Gewebes beurteilen zu können.

Das erfindungsgemäße Simulationsverfahren kann auch iterativ eingesetzt werden, wobei zum Beispiel in einem ersten Planungsschritt ausgehend von einem Anfangsdatensatz, zum Beispiel einer Anfangsposition eines Katheters und einer Anfangsdosis einer zu verabreichenden Substanz, in Verbindung mit einer vorgegebenen an einer oder mehreren bestimmten Stellen nach der Verabreichung der Substanz eingesetzten oder angelegten Energiemenge wie oben beschrieben berechnet oder simuliert wird, ob ein gewünschter Behandlungserfolg eintritt und ausgehend von dem Simulationsergebnis eine Modifikation des Behandlungsplanes durchgeführt wird, um einen möglicherweise besseren Behandlungserfolg zu erreichen. Diese Iteration kann so lange durchgeführt werden, bis ein optimales oder zum Beispiel oberhalb einer vorgegebenen Grenze liegendes Behandlungsresultat, wie zum Beispiel die Zerstörung von mindestens 80 % der Zellen eines Tumors, erreicht wird.

Weiterhin kann der mit dem oben beschriebenen Verfahren zur Planung der Verabreichung einer Substanz und von Energie ermittelte Parameter- oder Datensatz an ein bekanntes Navigationssystem übergeben werden, um das geplante Verfahren auszuführen Bei der Ausführung des Verfahrens ist es auch möglich zum Beispiel durch ein Kernspinresonanzverfahren den Ablauf der Behandlung zu überwachen, wie zum Beispiel in der US 6,026,316 beschrieben, und zum Beispiel zu überprüfen, ob der tatsächliche Behandlungsverlauf mit der geplanten Behandlung übereinstimmt, wobei im Falle einer Abweichung Infusionsparameter oder die Menge der verabreichten Energie verändert werden kann und zum Beispiel auch eine weitere Planung oder Simulation unter Verwendung der veränderten Parameter durchgeführt werden kann, um einen gewünschten Behandlungserfolg zu erreichen.

Nach der Durchführung der Verabreichung der Substanz in Kombination mit der Zufuhr von Energie kann das Ergebnis der Behandlung untersucht werden und Abweichungen von dem berechneten oder simulierten Ergebnis können verwendet werden, um bei einer weiteren Behandlung eine modifizierte Simulation durchzuführen.

Weiterhin bezieht sich die Erfindung auf ein Computerprogramm, welches, wenn es in einen Computer geladen ist, oder auf einem Computer läuft, ein wie oben beschriebenes Verfahren ausführt. Ebenso bezieht sich die Erfindung auf ein Programmspeichermedium oder ein Computerprogrammprodukt mit einem solchen Programm.

Eine Vorrichtung zur Planung der Verabreichung einer Substanz weist eine Dateneingabevorrichtung auf, welche zum Beispiel mit einer Datenerfassungseinheit, wie zum Beispiel einem Kernspintomographen oder Computertomographen, verbunden werden kann oder eine solche Einheit ist. Die eingegebenen Daten werden einer Recheneinheit bzw. einem Computer zugeführt, welcher in der Lage ist die eingegebenen Daten, wie zum Beispiel Bilddaten, auszuwerten und welcher weiterhin zum Beispiel aus einer Datenbank anatomische Daten zum Beispiel bezüglich der Stoffwechseleigenschaften oder der elektrischen Leitfähigkeit des zu behandelnden Gewebes erhält. Die Recheneinheit kann zum Beispiel einen herkömmlichen Computer und/oder ein neuronales Netzwerk und/öder eine Fuzzy-Logik enthalten, um aus einem Datensatz, wie zum Beispiel aus Messergebnissen einer Datenerfassungseinheit zur individuellen Untersuchung eines Körpers oder einer Gewebestruktur, patientenspezifische Parameter, wie zum Beispiel die elektrische oder thermische Leitfähigkeit des Gewebes oder eine Druckverteilung in dem Gewebe zu ermitteln. Weiterhin kann die Recheneinheit vorzugsweise die Verteilung der Substanz, insbesondere eine Fluidverteilung, eine Fluidkonzentration und/oder eine Energieverteilung simulieren unter Verwendung der Informationen, welche patientenspezifisch ermittelt wurden, wobei ergänzend allgemeine biologische oder anatomische Funktionen aus einer Datenbank, in welcher zum Beispiel ein generisches Modell abgespeichert ist, hinzugezogen werden können.

Vorzugsweise weist die Vorrichtung ein Navigationssystem und/oder eine Überwachungseinheit auf, mit welcher die Durchführung und/oder der Erfolg der Behandlung überprüft werden können, wobei zum Beispiel im Falle der Abweichung des tatsächlichen Verlaufs der Behandlung vom geplanten oder simulierten Verlauf der Behandlung eine oder mehrere Behandlungsparameter, wie zum Beispiel Infusions- oder Bestrahlungsparameter, modifiziert werden können.

Die Verwendung einer radioaktiven Flüssigkeit als ein Mittel zur Behandlung eines Gewebes (Brachytherapy seed) ist möglich, um im Gegensatz zur Verwendung von festen in den Körper eingebrachten Substanzen eine höhere Spezifität zu erreichen und ein vorgegebenes Zielvolumen genauer behandeln oder bestrahlen zu können. Insbesondere kann das Einbringen oder Verteilen der radioaktiven Flüssigkeit wie oben oder in der EP 1 316 324 der Anmelderin beschrieben durchgeführt werden, oder durch Füllen eines Ballons oder Ballonkatheters erfolgen.

Die Erfindung wird nachfolgend anhand eines Ausführungsbeispiels beschrieben. Es zeigen:
- Figur 1: ein Ablaufdiagramm einer Ausführungsform des erfindungsgemäßen Verfahrens; und
- Figur 2: eine Vorrichtung.

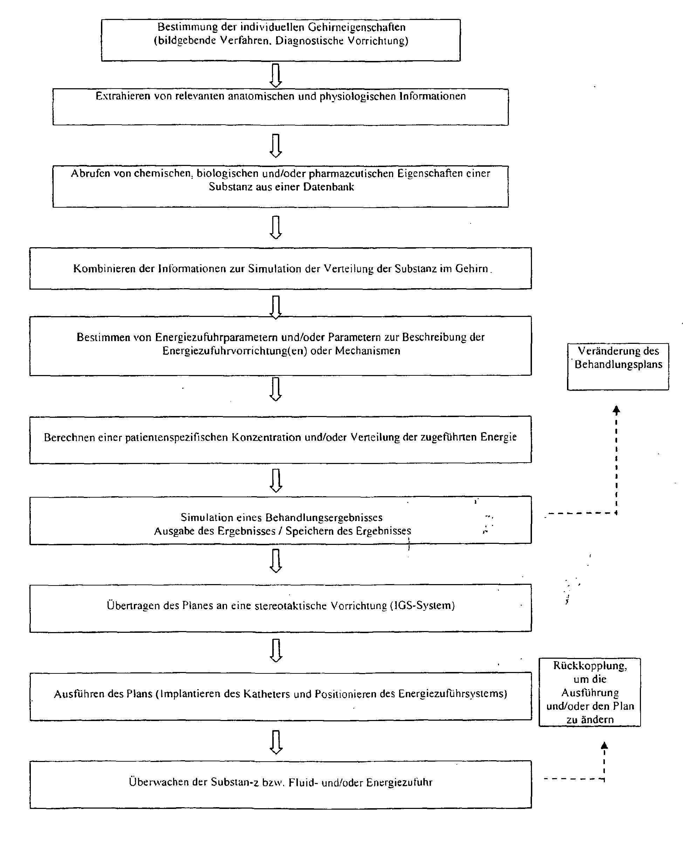

Figur 1 zeigt einen Ablaufplan für eine Ausführungsform des erfindungsgemäßen Verfahrens zur Behandlung eines Gehirntumors, wobei in einem ersten Schritt patientenspezifisch.die individuellen Gehirneigenschaften oder Gehirnparameter bestimmt werden, wozu zum Beispiel bekannte bildgebende Verfahren, insbesondere Kernspintomografie, Ultraschall, Computertomografie, PET, SPECT, Biopsie oder andere bekannte Verfahren verwendet werden können.

Die erfassten Daten zur Beschreibung von Eigenschaften des Gehirns werden anschließend ausgewertet, um relevante anatomische und/oder physiologische und/oder funktionelle Informationen zu erhalten. Hierzu können zum Beispiel ein generisches Modell oder auch bekannte mathematische Modelle zur Ermittlung von spezifischen für die Verteilung einer Substanz oder für die Absorption von Energie relevante Eigenschaften aus den erfassten Daten verwendet werden. Insbesondere können zum Beispiel das thermische Diffusionsvermögen, der Verlauf von Nervenbahnen, die Dichteverteilung, die Elastizität, der Flüssigkeitsgehalt, der Blutgehalt, Angaben zum Blutfluss, die vaskulare Permeabilität oder andere Informationen zur Beschreibung von Eigenschaften oder Strukturen des Gehirns verwendet werden. Werden zum Beispiel aus einem generischen Modell bekannte Muster oder allgemeine Informationen verwendet, so können Eigenschaften des Gehirns für bestimmte Volumen oder Gehirnbereiche abgeschätzt werden, wenn diese nicht direkt durch Messung ermittelt wurden.

Die durch eine Messung und/oder ein generisches Modell oder aus einer Datenbank ermittelten Informationen, welche für das erfindungsgemäße Verfahren relevant sein können, können eine oder mehrere der folgenden Mechanismen oder Eigenschaften sein:
- Transportmechanismus im Körper (zum Beispiel für Körperflüssigkeiten oder extern verabreichte Flüssigkeiten oder Substanzen)
- Abfluss oder Verteilung einer Flüssigkeit in dem Körper oder Gewebe (abhängig unter anderem von der Permeabilität oder von physiologischen Eigenschaften, wie beispielsweise der Blut-Hirn-Schranke)
- Hydraulische und/oder chemische und/oder biologische Eigenschaften an Körperstrukturgrenzen (Blut-Hirn-Schranke, Sulcii)
- Diffusion, welche sich auf den Transportmechanismus einer Substanz im Gewebe auswirkt
- Leitfähigkeit
- Grad der Energie-Sensitivität oder Energie-Empfindlichkeit
- Grad der Energie-Absorption
- Anatomische Information (weiße/graue Substanz)
   Wurde unter Verwendung von einer oder mehrerer der oben beschriebenen Eigenschaften oder Mechanismen ein Modell des Gehirns oder der zu behandelnden Körperregion zur Beschreibung des Transports oder der Verteilung einer Substanz oder Flüssigkeit, welche in das Gehirn oder die entsprechende Region eingebracht wird oder sich in dem Gehirn oder der entsprechenden Körperregion befindet, erstellt, so kann eine Verteilung einer zum Beispiel von extern in das Gehirn oder die zu behandelnde Körperregion eingebrachten Flüssigkeit in dem Gehirn oder der Körperregion simuliert werden, um eine lokale Verteilung und/oder Dosis zu erhalten, d.h. um zu ermitteln, welche absolute Menge oder Konzentration einer bestimmten Substanz sich in einem bestimmten Bereich oder einer bestimmten Struktur des Gewebes, wie zum Beispiel in einem bestimmten Gehirn- oder Körperbereich befindet. Zur Simulation können allgemeine chemische, biologische und/oder pharmazeutische Eigenschaften der verabreichten Substanz zum Beispiel aus einer Datenbank gewonnen werden, wobei ergänzend eine oder mehrere der folgenden Informationen oder Eigenschaften zur Simulation der Flüssigkeits- oder Substanzverteilung herangezogen werden können.
- Eine Information bezüglich der Verabreichungsparameter der Substanz (wie zum Beispiel die lokale Verabreichung in Gewebe oder die systemische Verabreichung; die Dauer der Verabreichung oder Infusion, die Auswirkung des Stoffwechsels (Metabolisation) auf die aktive Substanz oder den Trägermechanismus oder die Trägersubstanz, die Anordnung der Zuführvorrichtungen, (wie zum Beispiel Katheter und Pumpen)
- Gemessene oder zugewiesene bzw. bestimmte Eigenschaften des Zielbereiches oder - gewebes
- Pharmazeutische Eigenschaften des jeweiligen Mittels (wie zum Beispiel wärmeempfindliche Liposome, Energie oder Partikel emittierende Flüssigkeiten, Strahlungs-Sensitizer, Toxine, ...)
- mathematisches Modell, welches die oben erwähnten Informationen verwendet und mit dem sich eine Flüssigkeitsverteilung und/oder Dosis berechnen lässt

Weiterhin werden Parameter zur Energiezufuhr und Informationen bezüglich der Vorrichtung oder Mechanismen, mit welchen die Energie zugeführt wird, verwendet, um die Zufuhr von zum Beispiel Strahlungs-, Licht- oder Wärmeenergie in Gewebe zu simulieren. Zur Simulation der Energiezufuhr können eine oder mehrere der folgenden Informationen verwendet werden:
- gemessene oder zugeordnete Eigenschaften des Zielbereiches
- eine Information zur Anordnung oder Konzentration von Substanzen, Objekten oder Flüssigkeiten, welche sich auf die Energiesensitivitäts- und/oder die Absorptionsparameter des Zielbereiches auswirken
- eine Lageinformation der Energie emittierenden Objekte oder Flüssigkeiten
- Emissionseigenschaften der Objekte oder Flüssigkeiten
- Zeitparameter zur Objekt- oder Flüssigkeitsanordnung oder -Verteilung
- Energieverabreichungsparameter aus Quellen, welche außerhalb des Zielbereiches angeordnet sind
- ein mathematisches Modell, welches obige Informationen verwendet und eine lokale Verteilung der Energiezufuhr bestimmen kann.

Erfindungsgemäß werden die oben beschriebenen Simulationsergebnisse kombiniert, das heißt, es wird unter Berücksichtigung der simulierten Verteilung einer oder mehrerer Substanzen oder Flüssigkeiten in einem Gewebe oder einer Körperstruktur ermittelt, wie sich die simulierte Verabreichung von Energie gemäß einem Behandlungsplan zur zum Beispiel unterschiedlich dosierten Zufuhr von Energie in verschiedenen Körperbereichen auswirkt. Dabei kann zum Beispiel bestimmt werden, ob mit den zur Simulation verwendeten Daten des Behandlungsplanes, wie zum Beispiel dem Ort eines Katheters zum Einbringen einer Substanz und einer Abgabekennlinie der Substanz zum Beispiel zur Beschreibung des zeitlichen Verlaufs der abgegebenen Menge oder des Druckes, mit welcher die Substanz in das Gewebe eingebracht wird und unter Berücksichtigung der Mechanismen zur Zufuhr von Energie, zum Beispiel Tumorzellen in einer ausreichenden Menge zerstört werden können.

Die berechnete patientenspezifische Konzentration der eingebrachten Substanz und/oder die Verteilung der Substanz bzw. des Flüssigkeit und/oder die Energieverteilung werden erfindungsgemäß berechnet, wobei das Ergebnis ausgegeben und gespeichert werden kann.

Es ist möglich, die Reaktion des Zielbereiches über die Zeit zu simulieren, wobei auch die Parameter zur Beschreibung des Gewebeverhaltens über die Zeit verändert werden können, um das dynamische Verhalten des Zielbereiches zu beschreiben, so dass zum Beispiel auch durch die Behandlung auftretende Effekte oder Auswirkungen auf das Gewebe, zum Beispiel durch die verabreichte Substanz oder die zugeführte Energie für die Planung der Behandlung berücksichtigt werden können.

Ausgegeben werden kann zum Beispiel ein von einer Recheneinheit unter Verwendung des oben beschriebenen Verfahrens ermittelter Datensatz zum Beispiel in grafischer Darstellung, um eine oder mehrere der folgenden Informationen anzuzeigen:
- Verteilung der Substanzen und/oder Trägersubstanzen der Substanzen oder Medikamente
- lokal variierende Konzentration der Substanzen und/oder Trägersubstanzen
- Energieverteilung im Gewebe bei energieemittierenden Substanzen
- Energieverteilung einer externen Energiequelle
- Energieverteilung unter Berücksichtigung der Wirkung der verabreichten Flüssigkeiten oder Substanzen, welche die Energieabsorption und/oder Energieempfindlichkeit des Zielbereiches verändern (Wirkungsverteilung).
   Wird nach der Simulation festgestellt, dass ein gewünschtes oder vorgegebenes Behandlungsergebnis nicht oder nicht vollständig erreicht wird, so kann das Verfahren iterativ durchgeführt werden, wobei Behandlungsparameter, wie zum Beispiel die Dosierung oder Verabreichungsmechanismen der extern zugeführten Substanzen oder Flüssigkeiten oder die Mechanismen zur Zufuhr von Energie verändert werden und eine erneute Simulation mit den veränderten Ausgangsparametern durchgeführt wird, bis ein zufriedenstellendes Simulationsergebnis erhalten wird.

Ist das Simulationsergebnis zufriedenstellend, so kann der Behandlungsplan zum Beispiel an eine stereotaktische Vorrichtung übertragen werden, wie zum Beispiel ein bildunterstütztes Chirurgie (IGS)-System, um den Behandlungsplan auszuführen und um zum Beispiel einen oder mehrere Katheter an den durch die Simulation als günstig ermittelten Stellen zu platzieren.

Ergänzend kann während der Durchführung der Behandlung die Zufuhr der Flüssigkeit bzw. der Substanz und/oder der Energie gemessen oder überwacht werden, um dem Anwender eine Rückmeldung zu geben, ob der Behandlungsplan richtig ausgeführt wird oder geändert, werden sollte, so dass zum Beispiel Parameter zur Zufuhr der Substanz bzw. einer Flüssigkeit oder zur Zufuhr von Energie eingestellt oder abgeändert werden können, um ein besseres Behandlungsergebnis zu erreichen.

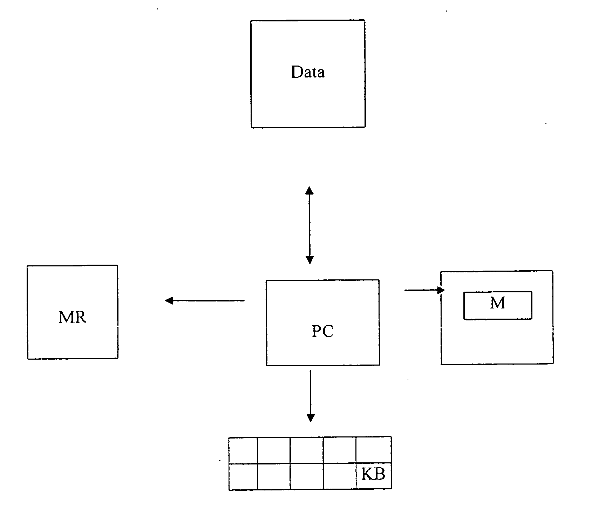

Figur 2 zeigt eine Vorrichtung, wobei ein Kernpinresonanztomograph MRI patentenspezifische Daten erfasst und an eine Recheneinheit PC ausgibt. Die Recheneinheit PC ist mit einer Datenbank DATA verbunden, in welcher ein generisches Modell des von dem Kernspintomographen MRI erfassten Körperbereiches abgelegt ist, wobei die Recheneinheit PC aus den von dem Kernspintomographen MRI und der Datenbank DATA übermittelten Daten ein Modell der Körperstruktur bildet, welches die patientenspezifischen Eigenschaften zur Verteilung einer der Körperstruktur zugeführten Substanz beschreibt.

Über eine Eingabeeinheit, wie zum Beispiel eine Tastatur KB, werden der Recheneinheit PC von einem Benutzer weitere Daten zum Beispiel bezüglich der Art einer zu verabreichenden Substanz oder der Art der zugeführten Energie eingegeben, wobei die Recheneinheit PC hierzu aus der Datenbank DATA ergänzende Informationen zum Beispiel bezüglich physikalischer, chemischer oder physiologischer Eigenschaften der Substanz abrufen kann, um das oben beschriebene Verfahren auszuführen. Die von der Recheneinheit PC ermittelten Simulationsergebnisse können an einem Monitor M grafisch dargestellt werden.

## Patentansprüche

1. Verfahren zur Planung der Behandlung von Gewebe durch die Verabreichung einer Substanz, welche Energie abgibt oder mit einer verabreichten Energie wechselwirken kann, an einen Körper, insbesondere an Körpergewebe, wobei eine Verteilung der Substanz in dem Körper simuliert wird und wobei das Verfahren iterativ durchgeführt wird, wenn das simulierte Ergebnis der Auswirkung der Verabreichung der Substanz oder Energie auf Gewebe unterhalb eines vorgegebenen Mindesteffektes liegt.

2. Verfahren nach Anspruch 1, wobei die Wechselwirkung der Substanz mit dem Gewebe simuliert wird.

3. Verfahren nach Anspruch 1, wobei zusätzlich basierend auf der simulierten Verteilung der Substanz im Körper eine Simulation der Verteilung einer durch die Substanz oder durch die Energiequelle zugeführten Energie durchgeführt wird, um zu Simulieren, wie sich die Kombination der Verabreichung der Substanz mit der Zufuhr der Energie auf den Körper oder einen Körper- oder Gewebebereich auswirkt.

4. Verfahren nach den Ansprüchen 1 bis 3, bei dem die in den Ansprüchen 1 bis 3 beschriebenen Verfahren beliebig miteinander kombiniert werden können.

5. Verfahren nach einem der vorherigen Ansprüche, wobei Körper- oder Gewebeeigenschaften, insbesondere physikalische, chemische oder physiologische Parameter eines oder mehrerer Gewebetypen ermittelt und bei der Simulation verwendet werden.

6. Verfahren nach einem der vorherigen Ansprüche, wobei die Auswirkung der Verabreichung der Substanz und der Zufuhr von Energie auf die Substanz in Verbindung mit dem Gewebe simuliert wird.

7. Verfahren nach einem der vorhergehenden Ansprüche, wobei simuliert wird, wie sich die Zufuhr einer externen Strahlungsenergie, Wärmeenergie, Kälteenergie und/oder Lichtenergie auf die verabreichte Substanz und/oder das Körpergewebe auswirkt.

8. Verfahren nach einem der vorhergehenden Ansprüche, wobei die zur Simulation verwendeten Parameter zur Beschreibung der Verabreichung der Substanz und zur Beschreibung der Verabreichung von Energie an ein Navigationssystem zur Ausführung des Verfahrens übertragen werden, wenn das Simulationsergebnis einen vorgegebenen Erfolg erzielt.

9. Verfahren nach einem der vorhergehenden Ansprüche, wobei die Auswirkung der Verabreichung der Substanz und/oder der Verabreichung der Energie untersucht wird und eine Neuplanung durchgeführt wird, falls eine Abweichung der tatsächlichen Behandlung von der simulierten Behandlung festgestellt wird.

10. Computerprogramm, welches, wenn es auf einem Computer läuft oder in einem Computer geladen ist, das Verfahren nach einem der vorhergehenden Ansprüche ausführt.

11. Programmspeichermedium oder Computerprogrammprodukt mit dem Programm nach dem vorhergehenden Anspruch.

## Claims

1. A method for planning to treat tissue by administering a substance which emits energy or can interact with administered energy to a body, in particular to body tissue, wherein a dispersion of the substance in the body is simulated and wherein the method is performed iteratively when the simulated result of the effect on tissue of administering the substance or energy is below a predetermined minimum effect.

2. The method according to claim 1, wherein the interaction of the substance with the tissue is simulated.

3. The method according to claim 1, wherein in addition, the distribution of energy supplied by the substance or by the energy source is simulated, based on the simulated dispersion of the substance in the body, in order to simulate the effect which the combination of administering the substance and supplying the energy has on the body or on an area of the body or tissue.

4. The method according to claims 1 to 3, wherein the methods described in claims 1 to 3 can be combined with each other in any way.

5. The method according to any one of the previous claims, wherein body or tissue characteristics, in particular physical, chemical or physiological parameters of one or more types of tissue are ascertained and used in the simulation.

6. The method according to any one of the previous claims, wherein the effect on the substance, in conjunction with the tissue, of administering the substance and supplying energy is simulated.

7. The method according to any one of the preceding claims, wherein the effect which supplying external radiation energy, heat energy, cooling energy and/or light energy has on the administered substance and/or the body tissue is simulated.

8. The method according to any one of the preceding claims, wherein the parameters used for the simulation, for describing administering the substance and for describing administering energy, are transferred to a navigation system for performing the method when the simulation result achieves a predetermined result.

9. The method according to any one of the preceding claims, wherein the effect of administering the substance and/or administering the energy is examined and the treatment is re-planned if a deviation of the actual treatment from the simulated treatment is established.

10. A computer program which, when it is running on a computer or is loaded onto a computer, performs the method according to any one of the preceding claims.

11. A program storage medium or computer program product comprising the program according to the preceding claim.

## Revendications

1. Procédé pour planifier le traitement d'un tissu par l'administration d'une substance dégageant de l'énergie ou pouvant interagir avec une énergie administrée, à un corps, en particulier à un tissu corporel, dans lequel une répartition de la substance dans le corps est simulée, et dans lequel le procédé est exécuté de façon itérative lorsque le résultat simulé de l'effet de l'administration de la substance ou de l'énergie sur le tissu se situe au-dessous d'un effet minimal prédéfini.

2. Procédé selon la revendication 1, dans lequel l'interaction de la substance avec le tissu est simulée.

3. Procédé selon la revendication 1, dans lequel, en plus d'être basé sur la répartition simulée de la substance dans le corps, une simulation de la répartition d'une énergie apportée par la substance ou par la source d'énergie est exécutée pour simuler comment la combinaison de l'administration de la substance avec l'apport en énergie affecte le corps ou une zone du corps ou du tissu.

4. Procédé selon les revendications 1 à 3, dans lequel les procédés décrits dans les revendications 1 à 3 peuvent être combinés librement entre eux.

5. Procédé selon l'une quelconque des revendications précédentes, dans lequel des propriétés d'un corps ou d'un tissu, en particulier des paramètres physiques, chimiques ou physiologiques d'un ou de plusieurs types de tissu sont déterminées et utilisées lors de la simulation.

6. Procédé selon l'une quelconque des revendications précédentes, dans lequel on simule l'effet de l'administration de la substance et de l'apport en énergie sur la substance en relation avec le tissu.

7. Procédé selon l'une quelconque des revendications précédentes, dans lequel on simule comment l'apport en énergie rayonnante, en énergie thermique, en énergie frigorifique et/ou en énergie optique externe(s) affecte la substance administrée et/ou le tissu corporel.

8. Procédé selon l'une quelconque des revendications précédentes, dans lequel les paramètres utilisés pour la simulation pour décrire l'administration de la substance et pour décrire l'administration d'énergie sont transmis à un système de navigation en vue d'exécuter le procédé lorsque le résultat de simulation obtient un succès prédéfini.

9. Procédé selon l'une quelconque des revendications précédentes, dans lequel l'effet de l'administration de la substance et/ou de l'administration de l'énergie est examiné et une nouvelle planification est effectuée si un écart est constaté entre le traitement réel et le traitement simulé.

10. Programme informatique, qui, lorsqu'il s'exécute sur un ordinateur ou lorsqu'il est chargé sur un ordinateur, exécute le procédé selon l'une quelconque des revendications précédentes.

11. Support de stockage de programme ou produit de programme informatique comprenant le programme selon la revendication précédente.
